# FASCICULE DE BREVET EUROPEEN

(11) **EP 0 714 276 B1**
(45) Date de publication et mention de la délivrance du brevet: **25.11.1998**
(21) Numéro de dépôt: 94924332.3
(22) Date de dépôt: 04.08.1994
(51) Int. Cl.: A61K 7/06, A61K 7/043

(54) **COMPOSITION CONTRE LA CHUTE DES CHEVEUX ET LA FRAGILITE DES ONGLES**
PRÄPARAT GEGEN HAARAUSFALL UND BRÜCHICHE NÄGEL
COMPOSITION AGAINST HAIR LOSS AND BRITTLE NAILS

(30) Priorité: 13.08.1993 FR 9309957
(43) Date de publication de la demande: 05.06.1996
(73) Titulaire: Oury, Jean-Pierre Maurice, 75008 Paris (FR)
(72) Inventeur: Oury, Jean-Pierre Maurice, 75008 Paris (FR)
(74) Mandataire: Bernasconi, Jean
(86) Numéro de dépôt international: FR9400979
(87) Numéro de publication internationale: WO9505146

(56) Documents cités:
- EP-A- 0 147 282
- EP-A- 0 382 619
- AU-A- 617 661
- SEIFEN-OELE-FETTE-WACHSE, vol.103, no.17, 27 Octobre 1977 pages 483 - 484 T. KUNZMANN 'Pflegemittel für die Nägel'

## Description

La présente invention a trait à une composition destinée à empêcher ou ralentir la chute des cheveux et également traiter les ongles fragiles et cassants.

Un grand nombre de compositions à administration topique ont déjà été proposées pour empêcher ou ralentir la chute des cheveux. Des régimes alimentaires ou des supplémentations ont également déjà été proposés. Les résultats sont généralement décevants.

Il existe donc le besoin d'une composition présentant une efficacité certaine et la présente invention se propose de fournir une telle composition, dont l'innocuité soit en même temps assurée.

L'invention a pour objet une composition à administration digestive pour le traitement des phanères, notamment contre la chute des cheveux et la fragilité des ongles, caractérisée en ce qu'elle contient une dose efficace d'inositol et d'un extrait sec de sésame.

La posologie journalière d'inositol est, de préférence, comprise entre 3 g et 300 mg. On préfère des posologies de 600 mg à 1 g environ. La composition est de préférence répartie en doses permettant d'atteindre ces quantités en une à trois prises par jour, de préférence au moment des repas.

La composition, administrée, de préférence par voie orale, peut comprendre, de préférence, en quantités efficaces, en outre un des éléments suivants et de façon préférée la plupart ou tous les éléments :
- extrait de millet
- spiruline ou algue isségué ou extraits d'algues équivalentes
- zinc (sulfate ou autre)
- magnésium (chlorure ou autre)
- et, de préférence, des acides aminés soufrés, de préférence DL-méthionine, et/ou L-cystine et/ou cystéine.

De préférence, la composition peut, en outre, comprendre d'autres éléments actifs, notamment des vitamines et des oligo-éléments. Parmi ces composants on préfère les suivants, seuls ou en combinaison :
- vitamine B6
- vitamine E
- acide pantothénique
- vitamine H
- acide folique
- vitamine B12
- levure de bière
- fer
- lithium (gluconate)
- biotine.

Ces éléments ont, d'une part, une action propre dans l'arrêt de la chute des cheveux, mais aussi d'autre part, une action favorisant l'effet des autres constituant de la composition.

Il est préféré que la composition contienne de l'inositol et au moins l'un des constituants : sésame, millet, spiruline ou isségué, et de préférence également du zinc et/ou du magnésium assimilables.

Par quantités efficaces, au sens de la présente invention, on entend des quantités qui soient, au minimum, d'un ordre de grandeur compatible avec les quantités qui sont données dans l'exemple détaillé ci-dessous, et, de préférence, supérieures au dixième de ces dernières. Une limite supérieure pour les quantités efficaces n'est pas indiquée car elle n'est pas critique, étant entendu qu'il faut évidemment éviter les surdosages, les seuils de surdosage étant d'ailleurs connus ou manifestement évidents pour les différents composants de la composition.

Par composition selon l'invention on entend également les compositions similaires dans lesquelles un ou quelques composants sont remplacés par des équivalents ayant sensiblement la même fonction.

L'inositol est un constituant de toutes les cellules aussi bien dans le règne animal que végétal. Sa formule chimique est identique à celle des sucres simples (C₆H₁₂O₆), mais les atomes sont disposés de manière différente. Une carence en inositol provoque chez les souris la perte de leurs poils, et les souriceaux voient leur croissance stoppée. Chez l'homme cela peut se traduire par l'apparition de calvitie.

Les extraits de sésame et de millet, de préférence sous forme d'extrait sec, et dont les fonctions sont complexes, favorisent la croissance des cheveux.

La spiruline ou l'isségué, obtenues à partir de l'algue, ont des effets similaires.

Le magnésium est un oligo-élément majeur dont la carence accélère le processus de vieillissement, l'atrophie du tissu conjonctif, la chute des cheveux.

Le zinc est un oligo-élement essentiel dans l'équilibre de l'organisme, qui joue un rôle important dans le métabolisme du glucose.

Un défaut en zinc provoque des troubles de la croissance (nanisme) ; les cheveux et les ongles poussent mal ; on peut observer des tâches blanches sous les ongles. Les cheveux se dépigmentent, blanchissent et deviennent fragiles et cassants. Un traitement au zinc favorise la repigmentation du cheveux.

Les acides aminés soufrés sont, de préférence, les suivants : D.L.-Méthionine, L(+)Cystéine HCL et L-Cystine, acides aminés soufrés qui constribuent à la croissance et à la formation des cheveux et des ongles.

Les vitamines sont nécessaires pour le métabolisme du folicule pileux. Les oligo-éléments qui agissent comme des catalyseurs et des régulateurs favorisent la vitalité et la pigmentation du cheveu, et l'élaboration des ongles. Enfin les acides aminés soufrés sont nécessaires à la formation du cheveu.

La vitamine B6, dont la pyridoxine en est la principale forme biochimique, est indispensable à l'ensemble du métabolisme des protéines et des acides aminés et à la synthèse des acides nucléiques.

Sa carence chez l'animal entraîne un arrêt du développement et l'apparition de troubles nerveux.

L'homme en hypovitaminose devient dépressif, et il peut présenter des signes de confusion mentale, de polynévrite, et de la séborrhée. De plus le processus de vieillissement est accéléré.

La vitamine C est un excellent régulateur du métabolisme.

Ses fonctions sont multiples : c'est la vitamine anti-infectieuse qui agit aussi sur le vieillissement cellulaire et sur les acides aminés. Elle active de nombreux enzymes et exerce un effet protecteur contre les carences des autres vitamines : A, B1, B2, D, E, acide pantothénique, biotine, acide folique.

La vitamine E est un anti-oxydant majeur pour la prévention du viellissement et la fécondité. On l'utilise traditionnellement dans des cas de stérilité, de troubles du tissu conjonctif, et de la cicatrisation de la peau.

L'acide pantothénique (B5) est un catalyseur très important. Il n'agit qu'en présence de biotine et d'acide folique. Il se trouve dans le lait, des fruits et légumes, la levure, les ovaires de poisson, les oeufs.

Sa carence chez le rat provoque un retard de croissance, une dépigmentation des poils, une inflammation de la peau.

La vitamine H ou biotine est une vitamine soufrée peu soluble. Sa carence chez l'animal se manifeste par une dégénérescence de la peau, du pelage, de la musculation. Chez l'homme on a pu observer une dermite desquamative séborrhéique, des parésthésies et une irritabilité nerveuse.

L'acide folique est transformé dans le foie de l'homme en sa forme active, l'acide folinique, en présence de vitamine C et de vitamine B12. Il joue un rôle important dans la croissance et la reproduction cellulaire. Avec la vitamine B1 il intervient dans la synthèse des acides nucléiques. Sa carence se manifeste par des troubles de la croissance et de l'ossification et des lésions de la peau et des muqueuses.

La vitamine B12 joue un rôle important dans la synthèse des acides aminés et elle est indispensable à l'élaboration des acides désoxyribonucléiques (ADN).

La levure de bière est particulièrement riche en vitamine H et combat le blanchissement du cheveux.

Le fer est un oligo-élément essentiel qui active les molécules d'oxygène, d'hydrogène et d'azote. Sa carence provoque, entre autres troubles, le blanchissement et la chute prématurée, des cheveux. De plus les ongles deviennent cassants et fragiles.

Le lithium, sous forme de traces, est choisi pour son action sur la perméabilité cellulaire et favorise l'entrée du magnésium et du calcium.

De préférence, la composition rassemble la totalité des éléments essentiels dans une unique formulation.

De préférence la formulation pour l'administration orale est effectuée sous forme de capsules ou de gélules.

La dose de composition par gélule est de préférence de l'ordre de 100 à 800 mg, et plus préférentiellement de 400 mg.

On va maintenant donner un exemple, non limitatif, de réalisation d'une composition selon l'invention, composition susceptible d'être contenue à l'intérieur d'une seule gélule.

| | |
|---|---|
| - inositol | 150 mg |
| - sésame extrait sec | 95 mg |
| - DL méthionine | 55 mg |
| - L cystéine HCL | 55 mg |
| - spiruline ou algue isségué | 40 mg |
| - levure de bière | 15 mg |
| - extrait de millet entier | 25 mg |
| - vitamine B6 | 5 mg |
| - vitamine C | 5 mg |
| - acide pantothénique (B5) | 1 mg |
| - vitamine H | 0,025 mg |
| - acide folique | 0,010 mg |
| - vitamine B12 | 0.001 mg |
| - L cystine | 5 mg |
| - chlorure de magnésium | 25 mg |
| - fer S.D. | 1 mg |
| - vitamine E | 3 mg |
| - sulfate de zinc | 3 mg |
| - lithium (gluconate) | 0,002 mg |

Tous les composants sont disponibles d'une façon générale dans le commerce.

Le traitement arrêtant ou supprimant la chute des cheveux en utilisant la composition selon l'invention, comporte, de préférence, un traitement d'attaque, par exemple deux mois, par exemple, par l'absorption de 3 gélules deux fois par jour au cours des repas. La phase d'entretien prévoit l'absorption de deux gélules deux fois par jour pendant au moins trois mois. Une cure de cinq mois est recommandée.
Un autre exemple est :

| FORMULE | |
|---|---|
| Inositol | 230 mg |
| Vitamine B6 | 0,2 mg |
| Vitamine C | 2,5 mg |
| Vitamine E | 1 mg |
| Acide pantothenique | 0,5 mg |
| Vitamine H | 0,015 mg |
| Acide folique | 0,025 mg |
| Levure de bière | 10 mg |
| Extrait de sésame | 45 mg |
| Extrait de millet entier | 10 mg |
| Spiruline | 24 mg |
| Gluconate de magnésium | 14,5 mg |
| Sulfate de zinc | 2,237 mg |
| Fer SD gluconate | 1 mg |
| DL méthionine | 28 mg |
| (+) cystéine HCl | 28 mg |
| cystéine | 3 mg |

Chaque gélule contient 400 mg.
Posologie : 4 gélules matin et midi, traitement de 3 mois.

## Revendications

1. Composition à usage oral pour le traitement des phanères, notamment contre la chute des cheveux et la fragilité des ongles, caractérisée en ce qu'elle contient une dose efficace d'inositol et d'un extrait sec de sésame.

2. Composition selon la revendication 1, caractérisée en ce qu'elle comprend en outre de l'extrait d'algue (spiruline ou isségué).

3. Composition selon la revendication 1, caractérisée en ce qu'elle comprend de l'inositol formulé en une dose efficace journalière de 300 mg à 3 g.

4. Composition selon l'une des revendications 1 à 3, caractérisée en ce qu'elle comprend, en quantités efficaces en outre un des éléments suivants :
- extrait de millet
- spiruline ou algue issegué ou extrait d'algues équivalentes
- zinc
- magnésium
- acides aminés soufrés, de préférence DL-méthionine et/ou L-cystine et/ou L-cystéine.

5. Composition selon la revendication 4, caractérisée en ce qu'elle comporte de l'inositol et du sésame, au moins l'un des constituants millet, spiruline ou isségué, et de préférence du zinc et/ou du magnésium.

6. Composition selon l'une des revendications 1 à 5, caractérisée en ce qu'elle comporte des vitamines et oligo-éléments.

7. Composition selon l'une des revendications 1 à 6, comprenant au moins l'un ou plusieurs, et de préférence la totalité des composés suivants :
- vitamine B6
- vitamine E
- acide pantothénique
- vitamine H
- acide folique
- vitamine B12
- levure de bière
- fer
- méthionine
- L-cystine
- biotine
- lithium (gluconate).

8. Composition selon la revendication 7, comprenant :
| | |
|---|---|
| - inositol | 150 mg |
| - sésame extrait sec | 95 mg |
| - DL méthionine | 55 mg |
| - L cystéine HCL | 55 mg |
| - spiruline ou algue isségué | 40 mg |
| - levure de bière | 15 mg |
| - extrait de millet entier | 25 mg |
| - vitamine B6 | 5 mg |
| - vitamine C | 5 mg |
| - acide pantothénique (B5) | 1 mg |
| - vitamine H | 0,025 mg |
| - acide folique | 0,010 mg |
| - vitamine B12 | 0.001 mg |
| - L cystine | 5 mg |
| - chlorure de magnésium | 25 mg |
| - fer S.D. | 1 mg |
| - vitamine E | 3 mg |
| - sulfate de zinc | 3 mg |
| - lithium (gluconate) | 0.002 mg |

9. Composition selon l'une des revendications 1 à 8, rassemblant la totalité des constituants essentiels dans une formulation unique.

10. Composition selon l'une des revendications 1 à 9, conditionnée sous forme de gélule.

11. Composition selon l'une des revendications 1 à 10, sous forme de dose de 100 à 800 mg, notamment 200 à 400 mg.

12. Composition selon l'une des revendications 1 à 11, caractérisée en ce qu'elle comprend :
| | |
|---|---|
| Inositol | 230 mg |
| Vitamine B6 | 0,2 mg |
| Vitamine C | 2,5 mg |
| Vitamine E | 1 mg |
| Acide pantothenique | 0,5 mg |
| Vitamine H | 0,015 mg |
| Acide folique | 0,025 mg |
| Levure de bière | 10 mg |
| Extrait de sésame | 45 mg |
| Extrait de millet entier | 10 mg |
| Spiruline | 24 mg |
| Gluconate de magnésium | 14,5 mg |
| Sulfate de zinc | 2,237 mg |
| Fer SD gluconate | 1 mg |
| DL méthionine | 28 mg |
| (+) cystéine HCl | 28 mg |
| cystéine | 3 mg |

## Claims

1. Composition for oral use for treatment of external structures secreted by the ectoderm, particularly for hair loss and brittle nails, characterised in that it contains an effective dose of inositol and of a dry extract of sesame.

2. Composition according to claim 1, characterised in that it comprises in addition extract of alga (spiruline or isségué).

3. Composition according to claim 1, characterised in that it comprises inositol formulated into an effective daily dose of 300 mg to 3 g.

4. Composition according to one of claims 1 to 3, characterised in that it comprises, in addition, effective quantities, one of the following elements:
- extract of millet
- spiruline of isségué alga or extract of equivalent algae
- zinc
- magnesium
- sulphurous amino acids, preferably DL-methionin and/or L-cystine and/or L-cysteine.

5. Composition according to claims 4, characterised in that it comprises inositol and sesame, at least one of the constituents millet, spiruline or isségué, and preferably zinc and/or magnesium.

6. Composition according to one of claims 1 to 5, characterised in that it comprises vitamins and oligo-elements.

7. Composition according to one of claims 1 to 6, comprising at least one or a plurality, and preferably all the following compounds:
- vitamin B6
- vitamin E
- pantothenic acid
- vitamin H
- folic acid
- vitamin B12
- beer yeast
- iron
- methionin
- L-cystine
- biotin
- lithium (gluconate).

8. Composition according to claim 7, comprising:
| | |
|---|---|
| - inositol | 150 mg |
| - dry sesame extract | 95 mg |
| - DL-methionin | 55 mg |
| - L-cysteine HCL | 55 mg |
| - spiruline or isségué alga | 40 mg |
| - beer yeast | 15 mg |
| - extract of whole millet | 25 mg |
| - vitamin B6 | 5 mg |
| - vitamin C | 5 mg |
| - pantothenic acid | 1 mg |
| - vitamin H | 0.025 mg |
| - folic acid | 0.010 mg |
| - vitamin B12 | 0.001 mg |
| - L-cystine | 5 mg |
| - magnesium chloride | 25 mg |
| - iron without solvent | 1 mg |
| - vitamin E | 3 mg |
| - zinc sulphate | 3 mg |
| - lithium (gluconate) | 0.002 mg |

9. Composition according to one of claims 1 to 8, containing all its essential constituents in a single formulation.

10. Composition according to one of claims 1 to 9, prepared in the form of a gel capsule.

11. Composition according to one of claims 1 to 10, in the form of a dose of 100 to 800 mg, particularly 200 to 400 mg.

12. Composition according to one of claims 1 to 11, characterised in that it comprises:
| | |
|---|---|
| inositol | 230 mg |
| vitamin B6 | 3.2 mg |
| vitamin C | 2.5 mg |
| vitamin E | 1 mg |
| pantothenic acid | 0.5 mg |
| vitamin H | 0.015 mg |
| folic acid | 0.025 mg |
| beer yeast | 10 mg |
| sesame extract | 45 mg |
| extract of whole millet | 10 mg |
| spiruline | 24 mg |
| magnesium gluconate | 14.5 mg |
| zinc sulphate | 2.237 mg |
| iron without solvent gluconate | 1 mg |
| DL-methionin | 23 mg |
| (-) cysteine HCL | 28 mg |
| cysteine | 3 g |

## Patentansprüche

1. Zusammensetzung zur oralen Verwendung bei der Behandlung von Exoskeletonen, insbesondere gegen Haarausfall und brüchige Nägel, dadurch gekennzeichnet, daß sie eine wirksame Dosis von Inositol und eines getrockneten Sesamextraktes enthält.

2. Zusammensetzung gemäß Anspruch 1, dadurch gekennzeichnet, daß sie außerdem Algenextrakt (Spirulin oder Isségué) umfaßt.

3. Zusammensetzung gemäß Anspruch 1, dadurch gekennzeichnet, daß sie Inositol umfaßt, das in einer täglichen wirksamen Dosis von 300 mg bis 3 g formuliert ist.

4. Zusammensetzung gemäß einem der Ansprüche 1 bis 3, dadurch gekennzeichnet, daß sie außerdem eine wirksame Menge der folgenden Elemente umfaßt:
- Hirseextrakt
- Spirulin oder Alge Issegué oder Extrakt äquivalenter Algen
- Zink
- Magnesium
- schwefelhaltige Aminosäuren, vorzugsweise DL-Methionin und/oder L-Cystin und/oder L-Cystein.

5. Zusammensetzung gemäß Anspruch 4, dadurch gekennzeichnet, daß sie Inositol und Sesam, wenigstens einen der Bestandteile Hirse, Spirulin oder Isségué und vorzugsweise Zink oder Magnesium umfaßt.

6. Zusammensetzung gemäß einem der Ansprüche 1 bis 5, dadurch gekennzeichnet, daß sie Vitamine und Spurenelemente umfaßt.

7. Zusammensetzung gemäß einem der Ansprüche 1 bis 6, dadurch gekennzeichnet, daß sie wenigstens einen oder mehrere und vorzugsweise die Gesamtheit der folgenden Bestandteile umfaßt:
- Vitamin B6
- Vitamin E
- Pantothensäure
- Vitamin H
- Folsäure
- Vitamin B12
- Bierhefe
- Eisen
- Methionin
- L-Cystin
- Biothin
- Lithium(gluconat)

8. Zusammensetzung gemäß Anspruch 7, dadurch gekennzeichnet, daß sie
| | |
|---|---|
| - Inositol | 150 mg |
| - getrockneter Sesamextrakt | 95 mg |
| - DL-Methionin | 55 mg |
| - L-Cystein HCl | 55 mg |
| - Spirulin oder Alge Isségué | 40 mg |
| - Bierhefe | 15 mg |
| - Hirseextrakt (gesamt) | 25 mg |
| - Vitamin B6 | 5 mg |
| - Vitamin C | 5 mg |
| - Pantothensäure (B5) | 1 mg |
| - Vitamin H | 0,025 mg |
| - Folsäure | 0,010 mg |
| - Vitamin B12 | 0,001 mg |
| - L-Cystin | 5 mg |
| - Magnesiumchlorid | 25 mg |
| - Eisen S.D. | 1 mg |
| - Vitamin E | 3 mg |
| - Zinksulfat | 3 mg |
| - Lithium(gluconat) | 0,002 mg |
umfaßt.

9. Zusammensetzung gemäß einem der Ansprüche 1 bis 8, dadurch gekennzeichnet, daß die Gesamtheit der wesentlichen Bestandteile in einer einzigen Formulierung zusammengestellt ist.

10. Zusammensetzung gemäß einem der Ansprüche 1 bis 9, dadurch gekennzeichnet, daß sie in Kapselform abgepackt ist.

11. Zusammensetzung gemäß einem der Ansprüche 1 bis 10 in einer Dosierung von 100 bis 800 mg, insbesondere 200 bis 400 mg.

12. Zusammensetzung gemäß einem der Ansprüche 1 bis 11, dadurch gekennzeichnet, daß sie
| | |
|---|---|
| Inositol | 230 mg |
| Vitamin B6 | 0,2 mg |
| Vitamin C | 2,5 mg |
| Vitamin E | 1 mg |
| Pantothensäure | 0,5 mg |
| Vitamin H | 0,015 mg |
| Folsäure | 0,025 mg |
| Bierhefe | 10 mg |
| Sesamextrakt | 45 mg |
| Hirseextrakt (gesamt) | 10 mg |
| Spirulin | 24 mg |
| Magnesiumgluconat | 14,5 mg |
| Zinksulfat | 2,237 mg |
| Eisen SD-gluconat | 1 mg |
| DL-Methionin | 28 mg |
| (⁺)-Cystein HCl | 28 mg |
| Cystein | 3 mg |
umfaßt.
